# EUROPEAN PATENT SPECIFICATION

(11) **EP 0 986 313 B1**
(45) Date of publication and mention of the grant of the patent: **06.09.2006**
(21) Application number: 98934911.3
(22) Date of filing: 04.06.1998
(51) Int. Cl.: A23K 1/00

(54) **STARCH-BASED PHOSPHATASE GRANULATES**
AUF STÄRKE BASIERTE PHOSPATASE-GRANULATE
GRANULES DE PHOSPHATASE A BASE D'AMIDON

(30) Priority: 04.06.1997 EP 97201641; 04.06.1997 US 48611 P
(43) Date of publication of application: 22.03.2000
(73) Proprietor: BASF Aktiengesellschaft, 67056 Ludwigshafen (DE)
(72) Inventor: BARENDSE, Rudolf, Carolus, Maria, NL-2613 CM Delft (NL); MEESTERS, Gabriel, Marinus, Henricus, NL-2614 TJ Delft (NL); HARZ, Hans-Peter, D-67373 Dubenhofen (DE)
(74) Representative: Maurer, Stefanie
(86) International application number: PCT/EP1998/003327
(87) International publication number: WO 1998/054980

(56) References cited:
- EP-A- 0 257 996
- EP-A- 0 304 332
- EP-A- 0 758 018
- WO-A2-98/26037
- DE-A- 3 344 104
- GB-A- 1 143 373
- US-A- 3 661 786
- PATENT ABSTRACTS OF JAPAN vol. 016, no. 106 (C-0919) 16 March 1992 & JP 03 280 839 A (NIPPON SODA CO LTD) 11 December 1991

## Description

### Field of the Invention

The present invention relates to the formulation of enzymes, preferably feed-enzymes, into starch containing granulates, and to processes for the preparation of such enzyme-containing granulates. These (edible) granulates can then be used in animal feeds.

### Background of the Invention

The use of various enzymes in animal, e.g. livestock, feed has become almost common practice. These enzymes are usually produced by culturing microorganisms in large scale fermenters operated by industrial enzyme producers. At the end of the fermentation the resulting "broth" is usually subjected to a series of filtration steps to separate the biomass (the microorganisms) from the desired enzyme (in solution). The enzyme solution is either then sold as a liquid (often after addition of various stabilizers) or processed to a dry formulation.

Enzyme liquid and dry formulations are used on a commercial scale by the feed industry. Liquid formulations may be added to the feed after pelleting in order to avoid heat inactivation of the enzyme (s) which would occur during the pelleting process. However the amounts of enzyme in the final feed preparations are usually very small which makes it difficult to achieve a homogenous distribution of the enzyme in the feed, and liquids are notoriously more difficult to mix evenly than dry ingredients. In addition one needs specialised (expensive) equipment to add liquids to the feed after pelleting which is not currently available at most feed mills (due to the extra cost).

Dry formulations of enzyme(s), on the other hand, have the disadvantage of heat-inactivation of the enzymes during pelleting. Preferred manufacturing protocols in the feed industry involve steam pelleting where the feed is subjected to steam injection(s) prior to pelleting. In the subsequent pelleting step the feed is forced through a matrix or die and the resulting strips are cut into suitable pellets of variable length. The moisture content immediately before pelleting is generally between 18% and 19%. During this process temperatures may rise to 60-95°C. The combined effect of high moisture content and high temperature is detrimental to most enzymes. These disadvantages are also encountered in other types of thermomechanical treatments such as extrusion and expansion.

In order to try and overcome these problems EP-A-0,257,996 suggests that the stability of enzymes in feed processing could be increased by the preparation of an enzyme "premix" where an enzyme-containing solution is absorbed onto a grain-based carrier consisting of flour, and the premix is subsequently pelleted and dried. However, these flour-based premixes are not suitable for gentler methods of processing (of the dough-like premix) into granulates, such as low-pressure extrusion or high shear granulation, because of the gluey character of the flour-baaed premixes.

Various enzyme manufacturers have developed alternative formulation methods to try to improve the stability of dry enzyme products during pelleting and storage.

EP-A-0,569,468 (Novo Nordisk) refers to a formulation consisting of a enzyme-containing "T-granulate" that is coated with a high melting wax or fat alleged to improve resistance to pelleting conditions. The granulate is prepared by mixing a dry inorganic (e.g. sodium sulphate) filler with the enzyme solution in a high shear granulator. EP-A-0,569,468 teaches that any beneficial effect of the coating with respect to pelleting stability is specific for the type of granulate coated, which in this case is based on a sodium sulphate filler. However, the absorption capacity of these (sodium sulphate) fillers is much less than that of carriers such as flour, which is undesirable if one wishes to produce more concentrated enzyme-containing granulates.

In addition, the granulates have a wide particle size distribution which makes it difficult to obtain a homogeneous enzyme concentration throughout. Moreover the bioavailability of the enzyme to the animal is decreased by the wax or fat coating.

EP 758 018 refers to salt stabilized spray dried solid enzyme preparations comprising inorganic salts and grain based carriers like wheat middlings or rice hulls.

WO-A-97/16076 (Novo Nordisk) also refers to the use of waxes and other water-insoluble substances in particulates but here they are employed as a matrix material.

There is thus a need for stable formulations of enzymes that are based on a carrier that is suitable for granulation methods other than pelleting and that can have a high absorption capacity.

### Description of the Invention

In a first aspect of the present invention there is provided a process for the preparation of a phosphatase containing granulate suitable for use in an animal feed, the process comprising processing a phosphatase, a solid carrier comprising at least 30% (w/w) of stands and water in appropriate relative amounts to obtain phosphatase containing granules with a water content of 30% to 40%, and subsequently drying the granules. The phosphatase containing granulate produceable by this process (which forms the second aspect of the invention, which also covers a granulate comprising dried granules formed from an enzyme and a solid carrier which comprises at least 30% (w/w) of starch seeks to solve or at least mitigates the problems encountered in the prior art.

The invention can thus provide processes for the preparation of enzyme formulations in the form of granulates that use the carbohydrate as a carrier. The carrier may be in particulate or powder form. The enzyme and water are preferably provided as an enzyme-containing (preferably aqueous) liquid, such as a solution or a slurry, which can be mixed with the solid carrier and allowed to absorb onto the carrier. During or after the mixing, the enzyme-containing liquid and the carrier are processed into a granulate, which can then subsequently be dried. The use of the carbohydrate carrier may allow the absorption of large amounts of enzyme-containing liquid (and therefore enzyme). The mixture may be used to form a plastic paste or non-elastic dough that can readily be processed into granules, for example it is extrudable. Suitably the carrier is non-fibrous which allows for easier granulation: fibrous materials can prevent granulation by extrusion.

A number of prior art documents that refer to pellets containing various enzymes, but these find use as detergents, often in washing compositions. In contrast, the present application finds use in animal feeds and for that reason the granulates of the invention are edible (by animals) and preferably also digestible. It will therefore not be surprising that the granulates, granules and compositions of the invention are free of soap, detergents and bleach or bleaching compounds, zeolites, binders, fillers (TiO₂, kaolin, silicates, talc etc) to name but a few.

The edible carbohydrate polymer should be chosen so that it is edible by the animal for whom the feed is intended, and preferably digestible as well. The polymer comprises glucose (e.g. a glucose-containing polymer), or (C₆H₁₀O₅)ₙ, units. The carbohydrate polymer comprises α-D-glucopyranose units, amylose (a linear (1→4) α-D-glucan polymer) and/or amylopectin (a branched D-glucan with α-D-(1→4) and α-D-(1→6) linkages). Starch is the selected carbohydrate polymer. Other suitable glucose-containing polymers that can be used in addition to starch, include α-glucans, β-glucans, pectin (such as proto-pectin), and glycogen. Derivatives of these carbohydrate polymers, such as ethers and/or esters thereof, are also contemplated although gelatinised starch is best avoided and thus may not be present. Suitably the carbohydrate polymer is water-insoluble.

In the examples described herein corn-, potato- and rice-starch is used. However, starch obtained from other (e.g. plant, such as vegetable or crop) sources such as tapioca, cassava, wheat, maize, sago, rye, oat, barley, yam, sorghum, or arrowroot is equally applicable. Similarly both native or modified (e.g. dextrin) types of starch can be used in the invention. Preferably the carbohydrate (e.g. starch) contains little or no protein, e.g. less than 5% (w/w), such as less than 2% (w/w) preferably less than 1% (w/w). Regardless of the type of starch (or other carboydrate polymer) it should be in a form that allows it to be used in an animal feed, in other words an edible or digestible form.

At least 30% (w/w) of the solid carrier comprises starch optimally at least 40% (w/w). Advantageously the major component of the solid carrier is the carbohydrate (e.g. starch), for example more than 50% (w/w), preferably at least 60% (w/w), suitably at least 70% (w/w), and optimally at least 80% (w/w). These weight percentages are based on the total weight of the non-enzymatic components in the final dry granulate.

In the process of the invention the enzyme and water may be present in the same composition before contacting the solid carrier. In this respect, one may provide an enzyme-containing aqueous liquid. This liquid may be a solution or a slurry that is from, or derived from, a fermentation process. This fermentation process will usually be one in which the enzyme is produced. The fermentation process may result in a broth which contains the microorganisms (which produce the desired enzyme) and an aqueous solution. This aqueous solution, once separated from the microorganisms (for example, by filtration) can be the enzyme-containing aqueous liquid used in the invention. Thus in preferred embodiments the enzyme-containing aqueous liquid is a filtrate.

The amount of enzyme-containing liquid (and so enzyme) that can be absorbed onto the carrier is usually limited by the amount of water that can be absorbed. For natural, granular, starch this can vary between 25 - 30% (w/w), without using elevated temperatures (that cause the starch to swell). In practice the percentage of enzyme liquid to be added to the carbohydrate will often be much larger than this because the enzyme containing liquid will usually contain a significant amount of solids. The enzyme solution can contain about 25% (w/w) solids, as a result of which the carbohydrate (starch) and enzyme solution can be mixed at a ratio of carbohydrate:enzyme solution of 0.5:1 to 2:1, e.g. 1.2:1 to 1.6:1, such as at a ratio of about 60% (w/w):40% (w/w), respectively. Preferably the amount of liquid added to the solid carrier is such that (substantially) all the water in the (aqueous) liquid is absorbed by the carbohydrate present in the solid carrier.

At elevated temperatures starch and other carbohydrate polymers can absorb much larger amounts of water under swelling. For this reason the carbohydrate polymer is desirably able to absorb water (or enzyme-containing aqueous liquids). For example, corn starch can absorb up to three times its weight of water at 60°C and up to ten times at 70°C. The use of higher temperatures in order to absorb a greater amount enzyme-containing liquid is thus contemplated by the present invention, and indeed is preferable especially when dealing with thermostable enzymes. For these enzymes therefore the mixing of the solid carrier and liquid (or enzyme and water) can be conducted at elevated temperatures (e.g. above ambient temperature), such as above 30°C, preferably above 40°C and optimally above 50°C. Alternatively or in addition the liquid may be provided at this temperature.

However, in general, non-swelling conditions at lower (e.g. ambient) temperatures are preferred. This may minimise activity loss arising from instability of (heat sensitive) enzymes at higher temperatures. Suitably the temperature during the mixing of the enzyme and water is from 20 to 25°C.

The mechanical processing used in the present invention for making the mixture of the enzyme, water (e.g. an enzyme-containing liquid) and the solid carrier into granules (in other words granulating) can employ known techniques frequently used in food, feed and enzyme formulation processes. This may comprise expansion, extrusion, spheronisation, pelleting, high shear granulation, drum granulation, fluid bed agglomeration or a combination thereof. These processes are usually characterised by an input of mechanical energy, such as the drive of a screw, the rotation of a mixing mechanism, the pressure of a rolling mechanism of a pelleting apparatus, the movement of particles by a rotating bottom plate of a fluid bed agglomerator or the movement of the particles by a gas stream, or a combination thereof. These processes allow the solid carrier (e.g. in the form of a powder), to be mixed with the enzyme and water, for example an enzyme-containing liquid (an aqueous solution or slurry), and so subsequently granulated.

Alternatively the solid carrier can be mixed with the enzyme (e.g. in a powder form) to which water, such as a liquid (or slurry) is then added (which can act as granulating liquid).

In yet a further embodiment of the invention the granulate (e.g. an agglomerate) is formed by spraying or coating the enzyme-containing liquid onto the carrier, such as in a fluid bed agglomerator. Here the resulting granules can include an agglomerate as can be produced in a fluid bed agglomerator.

Preferably the mixing of the enzyme-containing liquid and the solid carrier additionally comprises kneading of the mixture. This may improve the plasticity of the mixture in order to facilitate granulation (e.g. extrusion).

If the granulate is formed by extrusion this is preferably performed at low pressure. This may offer the advantage that the temperature of the mixture being extruded will not, or only slightly, increase. Low-pressure extrusion includes extrusion for example in a Fuji Paudal basket- or dome- extruder. Preferably extrusion does not result in the temperature of the material being extruded to rise above 40°C. The extrusion may naturally produce granules (the granules may break off after passage through a die) or a cutter may be employed.

The granules will have a water content of from 30 to 40%, such as from 33 to 37%. The enzyme content is preferably from 3 to 15, such as 5 to 12% (e.g. at least 50,000ppm).

The granules obtained can be subjected to rounding off (e.g. spheronisation), such as in a spheromiser, e.g. a MARUMERISER™ machine and/or compaction. The granules can be spheronised prior to drying since this may reduce dust formation in the final granulate and/or may facilitate any coating of the granulate.

The granules can then be dried, such as in a fluid bed drier or, in case of the fluid bed agglomeration, can be immediately dried (in the agglomerator) to obtain (solid dry) granulates. Other known methods for drying granules in the food, feed or enzyme industry can be used by the skilled person. Suitably the granulate is flowable.

The drying preferably takes place at a temperature of from 25 to 60°C, such as 30 to 50°C. Here the drying may last from 10 minutes to several hours, such as from 15 to 30 minutes. The length of time required will of course depend on the amount of granules to be dried, but as a guide this is from 1 to 2 seconds per kg of granules.

After drying the granules, the resulting granulate preferably has a water content of from 3 to 10%, such as from 5 to 9%.

A coating may be applied to the granulate to give additional (e.g. favoured) characteristics or properties, like low dust content, colour, protection of the enzyme from the surrounding environment, different enzyme activities in one granulate or a combination thereof. The granules can be coated with a fat, wax, polymer, salt, unguent and/or ointment or a coating (e.g. liquid) containing a (second) enzyme or a combination thereof. It will be apparent that if desired several layers of (different) coatings can be applied. To apply the coating(s) onto the granulates a number of known methods are available which include the use of a fluidised bed, a high shear granulator, a mixer granulator, or a Nauta-mixer.

In other embodiments additional ingredients can be incorporated into the granulate e.g. as processing aids, for further improvement of the pelleting stability and/or the storage stability of the granulate. A number of such preferred additives are discussed below.

Salts may be included in the granulate, (e.g. with the solid carrier or water). Preferably (as suggested in EP-A-0,758,018) inorganic salt(s) can be added, which may improve the processing and storage stability of the dry enzyme preparation. Preferred inorganic salts are water soluble. They may comprise a divalent cation, such as zinc (in particular), magnesium, and calcium. Sulphate is the most favoured anion although other anions resulting in water solubility can be used. The salts may be added (e.g. to the mixture) in solid form. However, the salt(s) can be dissolved in the water or enzyme-containing liquid prior to mixing with the solid carrier. Suitably the salt is provided at an amount that is at least 15% (w/w based on the enzyme), such as at least 30%. However, it can be as high as at least 60% or even 70% (again, w/w based on the enzyme). These amounts can apply either to the granules or to the granulate. The granulate may therefore comprise less than 12% (w/w) of the salt, for example from 2.5 to 7.5%, e.g. from 4 to 6%.

If the salt is provided in the water then it can be in an amount of from 5 to 30% (w/w), such as 15 to 25%.

Further improvement of the pelleting stability may be obtained by the incorporation of hydrophobic, gel-forming or slow dissolving (e.g. in water) compounds. These may be provided at from 1 to 10%, such as 2 to 8%, and preferably from 4 to 6% by weight (based on the weight of water and solid carrier ingredients). Suitable substances include derivatised celluloses, such as HPMC (hydroxy-propyl-methyl-cellulose), CMC (carboxy-methyl-cellulose), HEC (hydroxy-ethyl-cellulose); polyvinyl alcohols (PVA); and/or edible oils. Edible oils, such as soy oil or canola oil, can be added (e.g. to the mixture to be granulated) as a processing aid, although often it will be preferred that the granulate does not contain any hydrophobic substances (e.g. palm oil).

Preferably the granules have a relatively narrow size distribution (e.g. they are monodisperse). This can facilitate a homogeneous distribution of the enzyme in the granules and/or the enzyme granulate in the animal feed. The process of the invention tends to produce granulates with a narrow size distribution. However, if necessary, an additional step can be included in the process to further narrow the size distribution of the granules, such as screening. The size distribution of the granulate is suitably between 100 µm and 2000 µm, preferably between 200 µm and 1800 µm and optimally between 300 µm and 1600 µm. The granules may be of irregular (but preferably regular) shape, for example approximately spherical.

The water or enzyme-containing liquid may comprise one or more enzyme(s) and are usually of microbial origin, e.g. obtained from a microbial fermentation. Usually the enzyme will be in an active form (for example it may have catalytic or physiological activity). Preferably the liquid is in a concentrated form, such as an ultra-filtrate (UF), which may allow the production of a granulate with a desired activity level.

Suitable enzyme(s) are those to be included in animal feed which includes pet food. The granulate of the present invention comprises a phosphatase. The function of these enzymes is often to improve the feed conversion rate, e.g. by reducing the viscosity or by reducing the anti-nutritional effect of certain feed compounds. Feed enzymes (such as phytase) reduce the amount of compounds which are harmful to the environment in the manure.

Enzymes for these purposes are: phosphatases, such as phytases (both 3-phytases and 6-phytases) and/or acid phosphatases; carbohydrases, such as amylolytic enzymes and plant cell wall degrading enzymes of which include cellulases such as β-glucanases, hemicelluloses such as xylanases, or galactanases; peptidases, galactosidases, pectinases, esterases; proteases, preferably with a neutral and/or acidic pH optimum; and lipases, preferably phospholipases such as the mammalian pancreatic phospholipases A2.

Preferably, the enzyme does not include starch degrading enzymes (for example amylases). In some embodiments proteases may be excluded as these may cause harm if ingested.

With a phosphatase, such as a phytase, preferably the final granulate will have an activity of from 5,000 to 10,000 such as from 6,000 to 8,000, FTU/g. If the enzyme is a plant cell wall degrading enzyme, for example a cellulase, and in particular a hemicellulose such as xylanase, then the final granulate may have an activity of the enzyme ranging from 3,000 to 100,000, preferably 5,000 to 80,000, and optimally 8,000 to 70,000, EXU/g. If the enzyme is a cellulase, such as β-gluconase, then the final granulate can have an enzyme activity of from 500 to 15,000, preferably from 1,000 to 10,000, and optimally from 1,500 to 7,000, BGU/g.

The granules may comprise from 5 to 20, e.g. from 7 to 15% of the enzyme(s). The enzyme(s) may be naturally occurring or recombinant.

In addition to these enzymes the invention is equally applicable to polypeptides with other biological activities, such as antigenic determinants, for example that find use in vaccines and/or polypeptides engineered to have an increased content of essential amino acids, of which the biological activity may be sensitive to thermal inactivation, and the term "enzyme" as used herein is to be construed accordingly.

A preferred process according to the invention therefore comprises:
a. mixing the water, phosphatase and solid carrier comprising at least 30% (w/w) of starch for example mixing the solid carrier with an aqueous enzyme-containing liquid;
b. optionally kneading the resulting mixture;
c. granulating, for example by mechanical processing, the mixture in order to obtain enzyme-containing granules, for example by using a granulator or by extrusion;
d. optionally spheronising the granules;
e. drying the resultant granules to obtain an enzyme-containing granulate.

During the entire process one will aim to keep the maximum temperature to which the enzyme(s) are exposed to below 80°C.

The granulates of the invention are suitable for use in the preparation of an animal feed. In such processes the granulates are mixed with feed substances, either as such, or as part of a premix. The characteristics of the granulates according to the invention allows their use as a component of a mixture which is well suited as an animal feed, especially if the mixture is steam treated and subsequently pelleted. The dried granules may be visible or distinguishable in such pellets.

Thus a third aspect of the present invention relates to a process for the preparation of animal feed, or a premix or precursor to an animal feed, the process comprising mixing a granulate of the second aspect with one or more animal feed substances (e.g. seeds) or ingredients. This can then be sterilised, e.g. subjected to heat treatment. The resulting composition is then suitably processed into pellets.

A fourth aspect of the invention relates to a composition comprising a granulate of the second aspect, which composition is preferably an edible feed composition such as an animal feed. This composition is preferably in the form of pellets (there may be 1-5, e.g. 2-4 dried granules per pellet).

The composition can have a water content of from 10 to 20%, e.g. from 12-15%. The amount of enzyme(s) is suitably from 0.0005 to 0.0012%, such as at least 5ppm.

A fifth aspect relates to a process for promoting the growth of an animal, the process comprising feeding an animal with a diet that comprises a granulate of the second aspect or a composition of the fourth aspect. Here, the animal diet can include either the granulate itself, or the granulate present in a feed.

Suitably the composition comprises from 0.05 to 2.0, such as 0.3 to 1.0, optimally 0.4 to 0.6 FTU/g of a phosphatase, e.g. a phytase. A xylanase may be present at from 0.5 to 50, e.g. 1 to 40 EXU/g. Alternatively or in addition a cellulase may be present at from 09.1 to 1.0, e.g. 0.2 to 0.4 BGU/g.

A sixth aspect of the present invention relates to the use of the granulate of the second aspect in, or as a component of, an animal feed or for use in an animal diet.

Suitable animals include farm animals (pigs, poultry, livestock), non-ruminants or monogastric animals (pigs, fowl, poultry, marine animals such as fish), ruminants (bovine or ovine, e.g. cows, sheep, goats, deer, calves, lambs). Poultry includes chickens, hens and turkeys.

Preferred features and characteristics of one aspect of the invention are equally applicable to another *mutatis mutandis.*

The following Examples are presented merely to illustrate the invention, and are not intended, or to be construed as, being limiting.

### EXAMPLES

### General Materials and Methods

Extrusion tests were performed using a Fuji Paudal DG-L1 basket extruder, with screen openings of 1.0 mm, screen thickness 1.2 mm, operating speed of 70 rpm, and a current of 0.6 - 2.0 A.

The spheroniser was a Fuji Paudal Marumerizer QJ-400, with a charge volume of 3 litres, plate pitch of 3 mm, retention time of 45-200 seconds and rotating speed of 750 rpm.

The high shear granulation tests were conducted using a Lödige type high shear granulator FM20, with a chopper speed of 1500 rpm and a ploughshare speed of 100 rpm. Powder was placed in the granulator and the enzyme-containing liquid was sprayed on top. The resulting granulates were dried in fluid bed drier.

The enzyme solutions used were:
- an ultra-filtrate of an *Aspergillus* derived phytase with an activity of 16840 FTU/g, and a dry solids content of 22.4% (w/w) (Examples 1 to 7).
- an ultra-filtrate containing a *Trichoderma* derived mixture of endo-xylanase and β-glucanase activities of 12680 EXU/g and BGU/g, and a dry solids content of 20.6% (w/w) (Example 8).

Phytase activity was determined according to the procedure "ISL-method 61696" (manual vanadate assay). β-glucanase activity was determined according to the procedure "ISL-method 62170" (manual viscosimetric assay). Endo-xylanase activity was determined according to the procedure "ISL-method 62169" (manual viscosimetric assay). ISL-methods are obtainable on request from Gist-brocades, Food Specialties, Agri Ingredients Group, Wateringseweg 1, P.O. Box 1, 2600 MA, Delft, The Netherlands.

### EXAMPLE 1

### Preparation of corn starch-based enzyme granulate by kneading, extrusion, spheronisation and drying

An enzyme preparation was obtained by mixing and kneading a mixture of 60% (w/w) of corn starch with 40% (w/w) of an ultra-filtrate containing phytase. This mixture was extruded using the Fuji Paudal basket extruder to obtain a wet extrudate which was then spheronised in the MARUMERISER™ for one minute to obtain round particles of an average diameter of 780 µm. These particles were subsequently dried in a fluid bed drier for 20 minutes at a bed temperature of 40°C, and an inlet temperature of 75°C. Approximately 500kg of the granules were dried in 18 minutes. The thus obtained dry enzyme granulate had an activity of 6980 FTU/g.

### EXAMPLE 2

### Preparation of a corn starch-based enzyme granulate by high shear granulation and drying

The phytase ultra-filtrate and corn starch were mixed in a batch type high shear granulator of the Lödige type, with a batch size of 20 litres. The granulator was filled with 60% (w/w) of corn starch and 40% (w/w) ultra-filtrate was sprayed into the mixer during the mixing process. After addition of the ultra-filtrate (10 minutes) the granulator continued mixing for another 5 minutes to allow the particles to be formed and compacted. The thus obtained granules were dried in a fluid bed drier as in Example 1. The resulting granulate had an activity of 7420 FTU/g. The median diameter of the particles was 480 µm.

### EXAMPLE 3

### Preparation of a corn starch-based enzyme granulate by mixing, pelleting and drying

A mixture of 40% (w/w) of the phytase ultra-filtrate and 60% (w/w) of corn starch was prepared. The mixture was pelleted using a Schlütter Press type PP85, where the extrudates were cut off by rotating knives at the extruder head, with a die plate containing holes of 1 mm in diameter. The pellets were dried as in Example 1, resulting in a final product with an activity of 7460 FTU/g. The median diameter of the particles was 1080 µm.

### EXAMPLE 4

### Preparation of a potato starch-based enzyme granulate containing soy oil and MgSO₄, additions by mixing, kneading, pelleting and drying

In a mixer/kneader 30 kg of potato starch was added and 2.5 kg of Soy oil was mixed in. Subsequently the phytase ultra-filtrate was added containing MgSO₄.7H₂O (3.5 kg of MgSO₄.7H₂O was dissolved in 14 kg of ultra-filtrate). The product was mixed thoroughly in the kneader, then extruded and dried in a fluid bed drier as in Example 1. This resulted in a product of 5870 FTU/g.

### EXAMPLE 5

### Preparation of a rice starch-based enzyme granulate by mixing, kneading, extrusion spheronisation and drying

A mixture was prepared by mixing and kneading 62% (w/w) rice starch and 38% (w/w) of the phytase ultra-filtrate. This mixture was extruded using the Fuji Paudal basket extruder to obtain a wet extrudate which was then spheronised in the MARUMERISER™ for one minute to obtain round particles of an average diameter of 785 *µ*m. These particles were subsequently dried in a fluid bed drier as in Example 1. The final activity of the granulate was 7280 FTU/g.

### EXAMPLE 6

### Preparation of a corn starch-based enzyme granulate containing an HPMC addition by mixing, kneading extrusion spheronisation and drying

An enzyme preparation was obtained by kneading a mixture of 54% (w/w) of corn starch, 5% of HPMC (hydroxy-propyl-methyl-cellulose) and 41% (w/w) of a phytase ultra-filtrate. This mixture was extruded using the Fuji Paudal basket extruder to obtain a wet extrudate which was spheronised in the MARUMERISER™ for one minute to obtain round particles of an average diameter of 780 µm. These were subsequently dried in a fluid bed drier for 20 minutes at 40°C bed temperature, and 75°C inlet temperature. The thus obtained dry enzyme granulate had an activity of 8470 FTU/g.

### EXAMPLE 7

### Preparation of a corn starch-based enzyme granulate containing an HEC addition by mixing kneading extrusion spheronisation and drying

An enzyme preparation was obtained by mixing and kneading 54% (w/w) of corn starch, 5% (w/w) of HEC (hydroxy-ethyl-cellulose) with 41% (w/w) of the phytase ultra-filtrate. This mixture was extruded using the Fuji Paudal basket extruder to obtain a wet extrudate which was spheronised in the MARUMERISER™ for one minute to obtain round particles of an average diameter of 780 µm. These were subsequently dried in a fluid bed drier for 20 minutes at 40°C bed temperature, and 75°C inlet temperature. The thus obtained dry enzyme granulate had an activity of 8410 FTU/g.

### EXAMPLE 8 for illustrative purposes only

### Preparation of a corn starch-based enzyme granulate by high shear granulation and drying

In a batch type high shear granulator of the Lödige type, with a batch size of 20 litres, 60% (w/w) of corn starch was mixed with 40% (w/w) of the ultra-filtrate containing endo-xylanase and β-glucanase in the following manner. The granulator was filled with corn starch and the ultra-filtrate was sprayed into the mixer during the mixing process. After addition of the ultra-filtrate (10 minutes) the granulator was operated for another 5 minutes to allow the particles to be formed and compacted. The thus obtained granules were dried in a fluid bed drier as in Example 1. The resulting granulate had an activity of 13100 EXU/g and 5360 BGU/g.

### EXAMPLE 9

### Comparison of the pelleting stabilities

The different enzyme granulates of the invention were subjected to a pelleting trial and their pelleting stability was compared with those of the standard feed enzyme formulations. The pelleting trial consisted of mixing the enzyme (granulate) with a feed premix at 1000 ppm. This mixture was treated by injection of steam to give a temperature rise to 70°C, after which the mixture was pelleted in a pelleting machine to obtain the feed pellets, which were subsequently dried. This type of process is typical for the feed industry to obtain feed pellets.

For NATUPHOS™, a phytase containing formulation used as a standard, for comparison, was a mixture of wheat middlings with spray dried ultra-filtrate.

For NATUGRAIN™, an enzyme preparation containing β-glucanase and endo-xylanase, this is a fluid bed prepared granule, made by coating a salt core with an enzyme layer, applied by spraying the core with an ultra-filtrate.

Table 1 summarizes the results of the pelleting trials. It is apparent from Table 1 that the granulates made using a carbohydrate carrier gave improved pelleting yields when compared with standard formulations.

**Table 1: Results of the pelleting tests**

| Example Number | Enzyme activity of the granules | | Enzyme yield after pelleting at 70°C |
|---|---|---|---|
| | FTU/g | EXU/g BGU/g | % |
| Ex. 1 | 6980 | - | 54.9 |
| Ex. 2 | 7420 | - | 51.8 |
| Ex. 3 | 7460 | - | 62.8 |
| Ex. 4 | 5870 | - | 62.7 |
| Ex. 5 | 7280 | - | 54.7 |
| Ex. 6 | 8470 | - | 69.6 |
| Ex. 7 | 8410 | - | 67.3 |
| Ex. 8 | - | 13100 | 61.3 |
| | | 5360 | 25.8 |
| Standard NATUPHOS™ | 5250 | - | 29.8 |
| Standard NATUGRAIN™ | - | 8150 | 38.6 |
| | | 6030 | 10.4 |

It is clear from Table 1 that the type of granulation method, i.e. mechanical processing, is not critical to addressing the problems to be solved by the invention. Formulations using the carbohydrate polymer provided a much better pelleting stability as compared to the known NATUPHOS™ and NATUGRAIN™ formulations.

## Claims

1. A process for the preparation of a phosphatase -containing granulate suitable for use in an animal feed comprising:
a) making a mixture of a phosphatase and a solid carrier comprising at least 30 % (w/w) of starch and water,
b) mechanically processing the above mixture to obtain enzyme-containing granules with a water content of 30 % to 40 %, and
c) drying the granules.

2. A process according to Claim 1, wherein the phosphatase is a phytase and/or add phosphatase.

3. A process according to Claim 1 or 2, wherein the water and phosphatase are provided as an phosphatase-containing aqueous liquid, optionally wherein the liquid is a filtrate derived from a fermentation process resulting in production of the phosphatase.

4. A process according to any preceding Claim, wherein the resulting mixture is kneaded before granulation.

5. A process according to any preceding Claim, wherein the mechanical processing comprises extrusion, pelleting, high-shear granulation, expansion, fluid bed agglomeration or a combination thereof.

6. A process according to any preceding Claim, wherein the processing is extrusion performed at low pressure and/or in a basket- or dome-extruder and/or wherein the granules obtained are spheronised prior to drying.

7. A process according to any preceding Claim, wherein the range of size of the granulate is from 100 µm to 2000 µm, preferably 200 to 1800 µm and most preferably 300 to 1600 µm.

8. An enzyme containing granulate producible by a process as defined in any preceding Claim.

9. A granulate comprising dried granules formed from a phosphatase and a solid carrier which comprises at least 30 % (w/w) of starch, wherein the range of size of the granulate is from 100 µm to 2000 *µ*m, preferably 200 to 1800 µm and most preferably 300 to 1600 *µ*m.

10. A granulate according to Claim 9, wherein the phosphatase is a phytase and/or acid phosphatase.

11. A granulate according to any one of Claims 8 to 10, wherein the granules comprise at least one divalent cation and/or one or more hydrophobic, gel-forming or water insoluble compound(s).

12. A granulate according to Claim 11, wherein the hydrophobic, gel-forming or water insoluble compound comprises a derivatised cellulose, polyvinyl alcohol (PVA) or an edible oil.

13. A granulate according to Claim 12, wherein the derivatised cellulose is hydroxy-propyl-methyl-cellulose, carboxy-methyl-cellulose or hydroxy-ethyl-cellulose and/or the edible oil is soy oil or canola oil.

14. A process for the preparation of an animal feed, or a premix of precursor to an animal feed, the process comprising mixing a granulate as defined in any one of Claims 8 to 13 with one or more animal feed substance(s) or ingredient(s).

15. A process according to Claim 14 wherein the mixture of feed substance(s); and granulate is sterilized or treated with steam, pelletised and optionally dried.

16. A composition comprising a granulate as defined in any one of Claims 8 to 13.

17. A composition according to Claim 16, which is an edible feed composition and/or an animal feed.

18. A composition according to Claims 16 or 17 which comprises pellets of one or more feed substance(s) or ingredient(s) mixed with a granulate according to any one of Claims 8 to 13 optionally wherein the ratio of granulate : feed substance(s) or ingredient(s) is at least 1 g : 1 kilo.

19. A composition, which is an animal feed or a premix or precursor to an animal feed, preparable by a process according to Claim 14 or 15.

20. A process for promoting the growth of an animal, the process comprising feeding an animal with a diet that comprises either a granulate as defined in any one of Claims 8 to 13 or a composition as defined in any of Claims 16 to 19.

21. Use of a granulate defined in any one of Claims 8 to 13 in, or as a component of, an animal feed or for use in an animal diet.

## Patentansprüche

1. Verfahren zur Herstellung eines Phosphatase enthaltenden Granulats, das zur Verwendung in einem Tierfutter geeignet ist, bei dem man:
a) eine Mischung aus einer Phosphatase, einem festen Träger, der mindestens 30 Gew.-% Stärke enthält, und Wasser herstellt
b) die obige Mischung mechanisch zu enzymhaltigen Granulatkörnern mit einem Wassergehalt von 30 bis 40% verarbeitet und
c) die Granulatkörner trocknet.

2. Verfahren nach Anspruch 1, bei dem es sich bei der Phosphatase um eine Phytase und/oder saure Phosphatase handelt.

3. Verfahren nach Anspruch 1 oder 2, bei dem man das Wasser und die Phosphatase in Form einer phosphatasehaltigen wäßrigen Flüssigkeit bereitstellt, wobei es sich bei der Flüssigkeit gegebenenfalls um ein Filtrat aus einem Fermentationsverfahren, das zur Produktion der Phosphatase führt, handelt.

4. Verfahren nach einem der vorhergehenden Ansprüche, bei dem man die erhaltene Mischung vor dem Granulieren knetet.

5. Verfahren nach einem der vorhergehenden Ansprüche, bei dem die mechanische Verarbeitung Extrudieren, Pelletieren, Granulieren unter hochscherenden Bedingungen, Expandieren, Wirbelschichtagglomeration oder eine Kombination davon umfaßt.

6. Verfahren nach einem der vorhergehenden Ansprüche, bei dem es sich bei der Verarbeitung um eine Extrusion bei Niederdruck und/oder in einem Korbextruder oder einer Kuppelstrangpresse handelt und/oder die erhaltenen Granulatkörner vor dem Trocknen sphäronisiert werden.

7. Verfahren nach einem der vorhergehenden Ansprüche, bei dem die Größe des Granulats im Bereich von 100 bis 2000 *µ*m, vorzugsweise 200 bis 1800 *µ*m und ganz besonders bevorzugt 300 bis 1600 *µ*m liegt.

8. Enzymhaltiges Granulat, das nach einem Verfahren nach einem der vorhergehenden Ansprüche hergestellt werden kann.

9. Granulat, enthaltend getrocknete Granulatkörner aus einer Phosphatase und einem festen Träger, der mindestens 30 Gew.-% Stärke enthält, wobei die Größe des Granulats im Bereich von 100 bis 2000 µm, vorzugsweise 200 bis 1800 µm und ganz besonders bevorzugt 300 bis 1600 µm liegt.

10. Granulat nach Anspruch 9, bei dem es sich bei der Phosphatase um eine Phytase und/oder saure Phosphatase handelt.

11. Granulat nach einem der Ansprüche 8 bis 10, bei dem die Granulatkörner mindestens ein zweiwertiges Kation und/oder eine oder mehrere hydrophobe, gelbildende oder wasserunlösliche Verbindungen enthalten.

12. Granulat nach Anspruch 11, bei dem es sich bei der hydrophoben, gelbildenden oder wasserunlöslichen Verbindung um eine derivatisierte Cellulose, Polyvinylalkohol (PVA) oder ein genießbares Öl handelt.

13. Granulat nach Anspruch 12, bei dem es sich bei der derivatisierten Cellulose um Hydroxypropylmethylcellulose, Carboxymethylcellulose oder Hydroxyethylcellulose und/oder bei dem genießbaren Öl um Sojaöl oder Canolaöl handelt.

14. Verfahren zur Herstellung eines Tierfutters oder einer Vormischung oder Vorstufe für ein Tierfutter, bei dem man ein Granulat nach einem der Ansprüche 8 bis 13 mit einer oder mehreren Tierfuttersubstanzen oder einem oder mehreren Tierfutterbestandteilen vermischt.

15. Verfahren nach Anspruch 14, bei dem man die Mischung aus Futtersubstanz(en) und Granulat mit Wasserdampf sterilisiert oder behandelt, pelletiert und gegebenenfalls trocknet.

16. Zusammensetzung, die ein Granulat nach einem der Ansprüche 8 bis 13 enthält.

17. Zusammensetzung nach Anspruch 16, bei der es sich um eine genießbare Futterzusammensetzung und/oder ein Tierfutter handelt.

18. Zusammensetzung nach Anspruch 16 oder 17, die Pellets aus einer oder mehreren Futtersubstanzen oder -bestandteilen in Abmischung mit einem Granulat nach einem der Ansprüche 8 bis 13 enthält, wobei gegebenenfalls das Verhältnis von Granulat zu Futtersubstanz(en) oder bestandteil(en) mindestens 1 g: 1 kg beträgt.

19. Zusammensetzung, bei der es sich um ein Tierfutter oder eine Vormischung oder Vorstufe für ein Tierfutter handelt, die bzw. das nach einem Verfahren nach Anspruch 14 oder 15 hergestellt werden kann.

20. Verfahren zur Förderung des Wachstums eines Tiers, bei dem man an das Tier eine Nahrung verfüttert, die ein Granulat nach einem der Ansprüche 8 bis 13 oder eine Zusammensetzung nach einem der Ansprüche 16 bis 19 enthält.

21. Verwendung eines Granulats nach einem der Ansprüche 8 bis 13 in einem Tierfutter oder als Komponente eines Tierfutters oder zur Verwendung bei der Tierernährung.

## Revendications

1. Procédé de préparation d'un produit de granulation contenant de la phosphatase qui est approprié pour une utilisation dans un aliment pour animaux, comprenant :
a) une réalisation d'un mélange d'une phosphatase et d'un support solide comprenant au moins 30% (poids/poids) d'amidon et d'eau,
b) un traitement mécanique du mélange ci-dessus pour obtenir des granules contenant une enzyme avec une teneur en eau de 30 % à 40 %, et
c) un séchage des granules.

2. Procédé suivant la revendication 1, dans lequel la phosphatase est une phytase et/ou une phosphatase acide.

3. Procédé suivant la revendication 1 ou 2, dans lequel l'eau et la phosphatase sont prévues sous la forme d'un liquide aqueux contenant de la phosphatase, le liquide étant éventuellement un filtrat dérivé d'un processus de fermentation donnant lieu à une production de la phosphatase.

4. Procédé suivant l'une quelconque des revendications précédentes, dans lequel le mélange résultant est pétri avant granulation.

5. Procédé suivant l'une quelconque des revendications précédentes, dans lequel le traitement mécanique comprend une extrusion, une pelletisation, une granulation à haut cisaillement, une expansion, une agglomération en lit fluide ou une de leurs combinaisons.

6. Procédé suivant l'une quelconque des revendications précédentes, dans lequel le traitement est une extrusion effectuée à basse pression et/ou dans une extrudeuse à panier ou à dôme et/ou dans lequel les granules obtenus sont rendus sphériques avant le séchage.

7. Procédé suivant l'une quelconque des revendications précédentes, dans lequel la plage de tailles du produit de granulation est de 100 *µ*m à 2 000 *µ*m, de préférence de 200 à 1 800 *µ*m et très avantageusement de 300 à 1 600 *µ*m.

8. Produit de granulation contenant une enzyme qui peut être produit par un procédé tel que défini dans l'une quelconque des revendications précédentes.

9. Produit de granulation comprenant des granules séchés formés à partir d'une phosphatase et d'un support solide qui comprend au moins 30 % (poids/poids) d'amidon, la plage de tailles du produit de granulation étant de 100 µm à 2 000 µm, de préférence de 200 à 1 800 µm et très avantageusement de 300 à 1 600 µm.

10. Produit de granulation suivant la revendication 9, dans lequel la phosphatase est une phytase et/ou une phosphatase acide.

11. Produit de granulation suivant l'une quelconque des revendications 8 à 10, dans lequel les granules comprennent au moins un cation divalent et/ou un ou plusieurs composés hydrophobes, formateurs de gel ou insolubles dans l'eau.

12. Produit de granulation suivant la revendication 11, dans lequel le composé hydrophobe, formateur de gel ou insoluble dans l'eau comprend une cellulose convertie en dérivé, de l'alcool polyvinylique (PVA) ou une huile comestible.

13. Produit de granulation suivant la revendication 12, dans lequel la cellulose convertie en dérivé est de l'hydroxypropylméthylcellulose, de la carboxyméthylcellulose ou de l'hydroxyéthylcellulose et/ou l'huile comestible est de l'huile de soja ou de l'huile de canola.

14. Procédé de préparation d'un aliment pour animaux ou d'un prémélange ou précurseur d'un aliment pour animaux, le procédé comprenant un mélange d'un produit de granulation tel que défini dans l'une quelconque des revendications 8 à 13 à une ou plusieurs substances ou ingrédients d'aliment pour animaux.

15. Procédé suivant la revendication 14, dans lequel le mélange de substance(s) alimentaire(s) et de produit de granulation est stérilisé ou traité par de la vapeur, pelletisé et éventuellement séché.

16. Composition comprenant un produit de granulation tel que défini dans l'une quelconque des revendications 8 à 13.

17. Composition suivant la revendication 16, qui est une composition alimentaire comestible et/ou un aliment pour animaux.

18. Composition suivant la revendication 16 ou 17, qui comprend des pellets d'un ou plusieurs ingrédients ou substances alimentaires mélangés à un produit de granulation suivant l'une quelconque des revendications 8 à 13, le rapport produit de granulation/substance(s) ou ingrédient(s) alimentaire(s) étant éventuellement d'au moins 1 g/l kg.

19. Composition qui est un aliment pour animaux ou un prémélange ou précurseur d'un aliment pour animaux, préparable par un procédé suivant la revendication 14 ou 15.

20. Procédé pour favoriser la croissance d'un animal, le procédé comprenant une alimentation d'un animal par un régime qui comprend un produit de granulation tel que défini dans l'une quelconque des revendications 8 à 13 ou une composition telle que définie dans l'une quelconque des revendications 16 à 19.

21. Utilisation d'un produit de granulation défini dans l'une quelconque des revendications 8 à 13 dans un aliment pour animaux ou comme composant de celui-ci ou pour une utilisation dans un régime animal.
